# EUROPEAN PATENT SPECIFICATION

(11) **EP 4 333 971 B1**
(45) Date of publication and mention of the grant of the patent: **04.03.2026**
(21) Application number: 22722957.2
(22) Date of filing: 25.04.2022
(51) Int. Cl.: A61N 1/36, A61B 5/37, A61B 5/377, A61B 5/383, A61B 5/00, A61B 34/20, A61B 34/00, A61N 1/05, A61B 5/293

(54) **ELECTROPHYSIOLOGICALLY GUIDED DEEP BRAIN STIMULATION ELECTRODE PLACEMENT**
ELEKTROPHYSIOLOGISCH GEFÜHRTE ELEKTRODENPLATZIERUNG ZUR TIEFEN HIRNSTIMULATION
PLACEMENT D'ÉLECTRODE DE STIMULATION CÉRÉBRALE PROFONDE À GUIDAGE ÉLECTROPHYSIOLOGIQUE

(30) Priority: 03.05.2021 US 202163183288 P
(43) Date of publication of application: 13.03.2024
(73) Proprietor: Boston Scientific Neuromodulation Corporation, Valencia, CA 91355 (US)
(72) Inventor: MALEKMOHAMMADI, Mahsa, Sherman Oaks, California 91411 (US); BOKIL, Hemant, Cambridge, Massachusetts 02138 (US); STEINKE, G., Karl, Valencia, California 91354 (US)
(74) Representative: Vossius & Partner Patentanwälte Rechtsanwälte mbB
(86) International application number: PCT/US2022/026130
(87) International publication number: WO 2022/235447

(56) References cited:
- US-A1- 2011 295 142
- US-A1- 2013 218 232
- US-A1- 2014 316 217
- ESMAEILI V ET AL: "Classifying Depth of Anesthesia Using EEG Features, a Comparison", 2007 ANNUAL INTERNATIONAL CONFERENCE OF THE IEEE ENGINEERING IN MEDICINE AND BIOLOGY SOCIETY : [EMBC '07] ; LYON, FRANCE, 22 - 26 AUGUST 2007 ; [IN CONJUNCTION WITH THE BIENNIAL CONFERENCE OF THE SOCIÉTÉ FRANÇAISE DE GÉNIE BIOLOGIQUE ET MÉDICAL (SFGB, 22 August 2007 (2007-08-22), pages 4106 - 4109, XP031337118, ISBN: 978-1-4244-0787-3

## Description

### CLAIM OF PRIORITY

This application claims the benefit of U.S. Provisional Application No. 63/183,288, filed on May 3 , 2021.

### TECHNICAL FIELD

This document relates generally to medical devices and more particularly to a system that uses a biomarker to guide deep brain stimulation surgery under general anesthesia.

### BACKGROUND

Neurostimulation, also referred to as neuromodulation, has been proposed as a therapy for a number of conditions. Examples of neurostimulation include Spinal Cord Stimulation (SCS), Deep Brain Stimulation (DBS), Peripheral Nerve Stimulation (PNS), and Functional Electrical Stimulation (FES). Implantable neurostimulation systems have been applied to deliver such a therapy. An implantable neurostimulation system may include an implantable neurostimulator, also referred to as an implantable pulse generator (IPG), and one or more implantable leads each including one or more electrodes. The implantable neurostimulator delivers neurostimulation energy through one or more electrodes placed on or near a target site in the nervous system. An external programming device can be used to program the implantable neurostimulator with stimulation parameters controlling the delivery of the neurostimulation energy. For DBS, the target electrodes are placed at or near a target site in the brain. The present inventor has recognized a need for improvement in DBS surgery. Known neurostimulation systems are shown for example in US 2014/316217 A1 and US 2011/295142 A1.

The article by ESMAEILI ET AL, entitled "Classifying Depth of Anesthesia Using EEG Features, a Comparison" (2007 ANNUAL INTERNATIONAL CONFERENCE OF THE IEEE ENGINEERING IN MEDICINE AND BIOLOGY SOCIETY, 22 - 26 AUGUST 2007, pages 4106-4109, ISBN: 978-1-4244-0787-3) shows how EEG features are used for assessing depth of anesthesia.

US 2013/218232 A1 relates a technique of electrical stimulation to the brain using a suppression window determined using a suppression threshold and an after-discharge threshold.

### SUMMARY OF THE INVENTION

The present invention is defined by the appended claims and provides a medical system according. to claim 1 and a method of operating a medical system as defined in claim 12.

Methods of stimulation and methods involving surgical steps are not presently claimed and are only shown hereinafter for better understanding the present invention.

### SUMMARY OF THE DISCLOSURE

In DBS, electrical neurostimulation therapy is delivered to implantable electrodes located at certain neurostimulation targets in the brain to treat neurological or neurophysiological disorders. Sensed neural electrical signals can be used as a biomarker of the depth of anesthesia during DBS surgery and as an aid for DBS electrode placement during surgery.

Example 1 can include subject matter (such as a medical system for coupling to one or more implantable electrodes) comprising a sensing circuit configured to sense one or more neural signals representative of neural activity of a subject when connected to an implantable electrode of the one or more implantable electrodes, a memory to store a reference signal, wherein the reference signal is representative of a neural response associated with a state of arousal at or near a location of the implantable electrode, and processing circuitry. The processing circuitry is configured to compare the one or more sensed neural signals to the reference signal, and determining a depth of anesthesia of the subject according to the comparison of the one or more sensed neural signals and the reference signal.

In Example 2, the subject matter of Example 1 optionally includes a stimulation circuit configured to deliver electrical neurostimulation to initiate an evoked neural signal. The sensing circuit is optionally configured to sense the evoked neural signal at or near the location of the implantable electrode, and the reference signal is a baseline evoked resonant neural activity signal for a specific anatomical location of the subject.

In Example 3, the subject matter of one or both of Examples 1 and 2 optionally includes a stimulation circuit configured to deliver electrical neurostimulation to initiate an evoked neural signal. The sensing circuit is configured to sense a local evoked neural signal at or near a location of the electrical neurostimulation, and the reference signal is a baseline local evoked neural signal for a specific anatomical location of the subject.

In Example 4, the subject matter of one or any combination of Examples 1-3 optionally includes a stimulation circuit configured to deliver electrical neurostimulation to initiate an evoked neural signal. The sensing circuit is configured to sense a network evoked neural signal using the implantable electrode, and the reference signal is a baseline network evoked neural signal for a specific anatomical location of the subject.

In Example 5, the subject matter of one or any combination of Examples 1-4 optionally includes an implantable electrode configured for placement at or near a basal ganglia structure of the subject.

In Example 6, the subject matter of one or any combination of Examples 1-5 optionally includes a sensing circuit configured to sense a spontaneous neural activity signal, and the reference signal is a baseline spontaneous neural activity signal associated with the state of arousal.

In Example 7, the subject matter or one or any combination of Examples 1-6 optionally includes a therapy circuit configured to control dosing of the subject with a general anesthesia drug, and processing circuitry configured to adjust delivery of the general anesthesia drug according to the indication of depth of anesthesia of the subject.

In Example 8, the subject matter of one or any combination of Examples 1-7 optionally includes memory that stores an anesthesia response map that contains reference signals stored in association with anatomical locations and with one or more of a type of general anesthetic drug and a dose of a general anesthetic drug. The processing circuity is optionally configured to compare the one or more sensed neural signals to the reference signal associated with an anatomical location of the implantable electrode and generate the indication of depth of anesthesia of the subject using the comparison to the reference signal, and the one or more of the type of general anesthetic drug and the dose of general anesthetic drug.

In Example 9, the subject matter of one or any combination of Examples 1-8 optionally includes processing circuitry configured to determine a measure of association of one or more time domain features of the one or more sensed neural signals to one or more time domain features of the reference signal, and generate the indication of depth of anesthesia of the subject according to the determined correlation.

In Example 10, the subject matter of one or any combination of Examples 1-9 optionally includes processing circuitry configured to determine a measure of association of one or more frequency domain features of the one or more sensed neural signals to one or more frequency domain features of the reference signal and generate the indication of depth of anesthesia of the subject according to the determined correlation.

In Example 11, the subject matter of one or any combination of one or any combination of Examples 1-10 optionally includes processing circuitry configured to perform a machine learning algorithm to classify the one or more sensed neural activity signals as matching the reference signal.

Example 12, includes subject matter (such as a method of operating a medical system), or can optionally be combined with one or any combination of Examples 1-11 to include such subject matter, comprising sensing one or more neural signals representative of neural activity of a subject using the medical system; comparing the one or more sensed neural signals to a reference signal using the medical system, wherein the reference signal is representative of a neural response associated with a state of arousal at or near an anatomical location of an implantable electrode; and determining a depth of anesthesia of the subject according to the comparison of the one or more sensed neural signals and the reference signal.

In Example 13, the subject matter of Example 12 optionally includes comparing the one or more sensed neural signals to multiple stored reference signals, each reference signal associated with an anatomical location and representative of a local and network response to general anesthesia at or near the anatomical location; matching the one or more sensed neural signals to a reference signal of the multiple stored reference signals; and generating the indication includes generating an indication of the anatomical location corresponding to the reference signal as the location of the implantable electrode.

In Example 14, the subject matter of Example 13 optionally includes receiving a target anatomical location via a user interface of the medical system, matching the one or more sensed neural signals to a reference signal for the target location, and generating an indication via the user interface that the implantable electrode is positioned at the target anatomical location.

In Example 15, the subject matter of one or both of Examples 13 and 14 optionally includes receiving a target anatomical location via a user interface of the medical system, determining an anatomical location of the implantable electrode by matching the one or more sensed neural signals to the reference signal, and indicating relative position of the implantable electrode and the target anatomical location via the user interface.

In Example 16, the subject matter of one or any combination of Examples 13-15 optionally includes receiving one or both of a type of anesthesia drug and a dose of the anesthesia drug via a user interface of the medical system, searching the stored multiple reference signals according to the one or both of the type of anesthesia drug and the dose of the anesthesia drug, and comparing the one or more sensed neural signals only to those reference signals of the multiple reference signals corresponding to the one or both of the type of anesthesia drug and the dose of the anesthesia drug.

In Example 17, the subject matter of one or any combination of Examples 13-16 optionally includes the one or more sensed neural signals includes an evoked neural signal and the method includes initiating the evoked neural signal using the medical system.

Example 18 includes subject matter (such as a medical system for coupling to one or more implantable electrodes) or can optionally be combined with one or any combination of Examples 1-17 to include such subject matter, comprising a sensing circuit configured to sense one or more neural signals representative of neural activity of a subject when connected to an implantable electrode of the one or more implantable electrodes, a memory to store multiple reference signals, each reference signal associated with a specific anatomical location and representative of a response to a general anesthesia drug at or near the specific anatomical location, and processing circuitry in communication with the sensing circuit and the memory. The processing circuitry is configured to compare the one or more sensed neural signals to the multiple reference signals, match the one or more sensed neural signals to a stored reference signal, and determining a specific anatomical location corresponding to the reference signal as the location of the implantable electrode.

In Example 19, the subject matter of Example 18 optionally includes a user interface and processing circuitry configured to receive a target anatomical location via the user interface, determine a reference signal for the target anatomical location, and generate an indication via the user interface that the implantable electrode is positioned at the target anatomical location.

In Example 20, the subject matter of one or both of Examples 18 and 19 optionally includes a user interface and processing circuitry configured to receive information of one or both of a type of anesthesia drug and a dose of the anesthesia drug via the user interface, search the stored multiple reference signals according to the one or both of the type of anesthesia drug and the dose of the anesthesia drug, and compare the one or more sensed neural signals to those reference signals of the multiple reference signals corresponding to the one or both of the type of anesthesia drug and the dose of the anesthesia drug.

These non-limiting examples can be combined in any permutation or combination. This summary is intended to provide an overview of subject matter of the present patent application. It is not intended to provide an exclusive or exhaustive explanation of the disclosure. The detailed description is included to provide further information about the present patent application. Other aspects of the disclosure will be apparent to persons skilled in the art upon reading and understanding the following detailed description and viewing the drawings that form a part thereof, each of which are not to be taken in a limiting sense.

### BRIEF DESCRIPTION OF THE DRAWINGS

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.
Figure 1 is an illustration of portions of an example of an electrical stimulation system.
Figures 2A-2C illustrate additional examples of electrical stimulation systems.
Figure 3 is a schematic side view of an example of an electrical stimulation lead.
Figures 4A-3H are illustrations of different embodiments of leads with segmented electrodes.
Figure 5 is an illustration representing deep brain stimulation electrode implantation.
Figures 6A-6C are illustrations of examples of spontaneous and evoked neural signal waveforms.
Figure 7 is a block diagram of portions of an example of portions of a medical device.
Figure 8 is a flow diagram of an example of a method of deep brain stimulation implantable lead placement.

### DETAILED DESCRIPTION

In the following detailed description, reference is made to the accompanying drawings which form a part hereof, and in which is shown by way of illustration specific embodiments in which the invention may be practiced. These embodiments are described in sufficient detail to enable those skilled in the art to practice the invention, and it is to be understood that the following detailed description provides examples, and the scope of the present invention is defined by the appended claims.

This document discusses devices, systems and methods for programming and delivering electrical neurostimulation to a patient or subject. Advancements in neuroscience and neurostimulation research have led to a demand for delivering complex patterns of neurostimulation energy for various types of therapies. The present system may be implemented using a combination of hardware and software designed to apply any neurostimulation (neuromodulation) therapy, including but not being limited to DBS therapy.

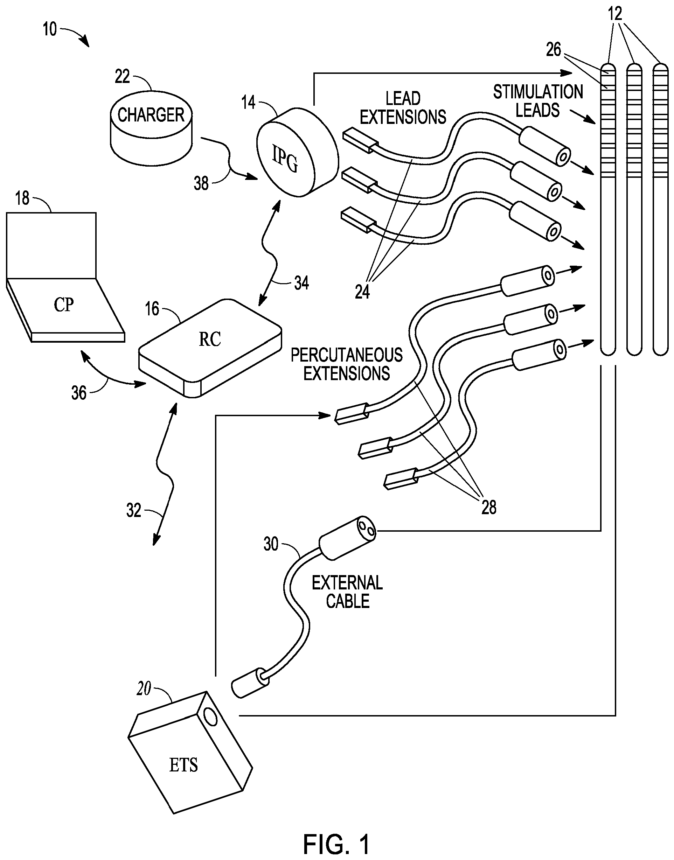

Figure 1 is an illustration of portions of an embodiment of an electrical stimulation system 10 includes one or more stimulation leads 12 and an implantable pulse generator (IPG) 14. The system 10 can also include one or more of an external remote control (RC) 16, a clinician's programmer (CP) 18, an external trial stimulator (ETS) 20, or an external charger 22. The IPG 14 can optionally be physically connected via one or more lead extensions 24, to the stimulation lead(s) 12. Each lead carries multiple electrodes 26 arranged in an array. The IPG 14 includes pulse generation circuitry that delivers electrical stimulation energy in the form of, for example, a pulsed electrical waveform (i.e., a temporal series of electrical pulses) to the electrode array 26 in accordance with a set of stimulation parameters. The IPG 14 can be implanted into a patient's body, for example, below the patient's clavicle area or within the patient's buttocks or abdominal cavity. The implantable pulse generator can have multiple stimulation channels (e.g., 8 or 16) which may be independently programmable to control the magnitude of the current stimulus from each channel. The IPG 14 can have one, two, three, four, or more connector ports, for receiving the terminals of the leads 12.

The ETS 20 may also be physically connected, optionally via the percutaneous lead extensions 28 and external cable 30, to the stimulation leads 12. The ETS 20, which may have similar pulse generation circuitry as the IPG 14, can also deliver electrical stimulation energy in the form of, for example, a pulsed electrical waveform to the electrode array 26 in accordance with a set of stimulation parameters. One difference between the ETS 20 and the IPG 14 is that the ETS 20 is often a non-implantable device that is used on a trial basis after the neurostimulation leads 12 have been implanted and prior to implantation of the IPG 14, to test the responsiveness of the stimulation that is to be provided. Any functions described herein with respect to the IPG 14 can likewise be performed with respect to the ETS 20.

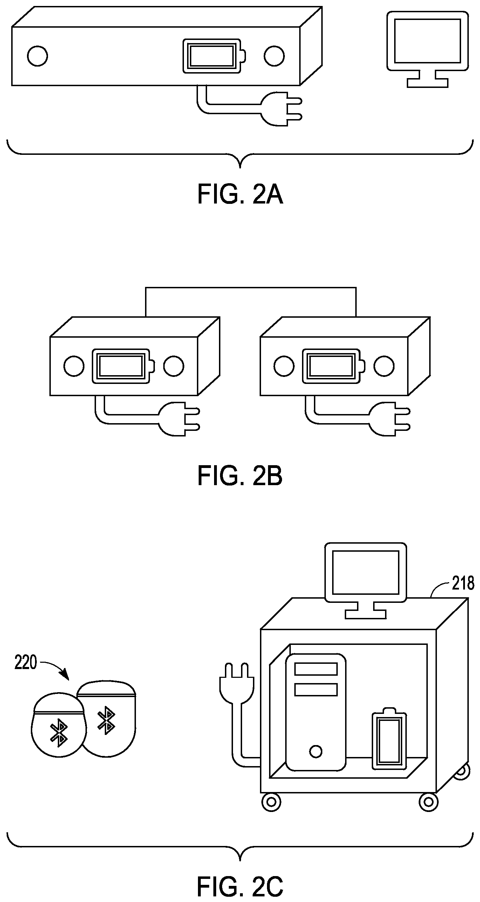

Figures 2A-2C illustrate examples of additional hardware configurations. In Figure 2A, the electrical stimulation hardware, the sensing hardware, and a user interface are included in the same unit. In Figure 2B, the electrical stimulation hardware and the sensing hardware are provided as separate units. The unit or units of Figures 2A and 2B can be capital equipment and can be rack mounted. The units shown in Figures 2A and 2B are wall powered but the units can be battery powered. Figure 2C illustrates an example that includes an ETS 220 and separate computer 218. The computer 218 communicates wirelessly with the ETS 220 (e.g., using Bluetooth hardware and communication protocol).

Returning to Figure 1, the RC 16 may be used to telemetrically communicate with or control the IPG 14 or ETS 20 via a wireless communications link 32. Once the IPG 14 and neurostimulation leads 12 are implanted, the RC 16 may be used to telemetrically communicate with or control the IPG 14 via communications link 34. The communication or control allows the IPG 14 to be turned on or off and to be programmed with different stimulation parameter sets. The IPG 14 may also be operated to modify the programmed stimulation parameters to actively control the characteristics of the electrical stimulation energy output by the IPG 14. The CP 18 allows a user, such as a clinician, the ability to program stimulation parameters for the IPG 14 and ETS 20 in the operating room and in follow-up sessions. The CP 18 may perform this function by indirectly communicating with the IPG 14 or ETS 20, through the RC 16, via a wireless communications link 36. Alternatively, the CP 18 may directly communicate with the IPG 14 or ETS 20 via a wireless communications link (not shown). The stimulation parameters provided by the CP 18 are also used to program the RC 16, so that the stimulation parameters can be subsequently modified by operation of the RC 16 in a stand-alone mode (i.e., without the assistance of the CP 18).

For purposes of brevity, the details of the RC 16, CP 18, ETS 20, and external charger 22 will not be further described herein. Details of exemplary embodiments of these devices are disclosed in U.S. Pat. No. 6,895,280, which is incorporated herein by reference. Other embodiments of electrical stimulation systems can be found at U.S. Patents Nos. 6,181,969; 6,516,227; 6,609,029; 6,609,032; 6,741,892; 7,949,395; 7,244,150; 7,672,734; 7,761,165; 7,974,706; 8,175,710; 8,224,450; 8,364,278; and 8,700,178.

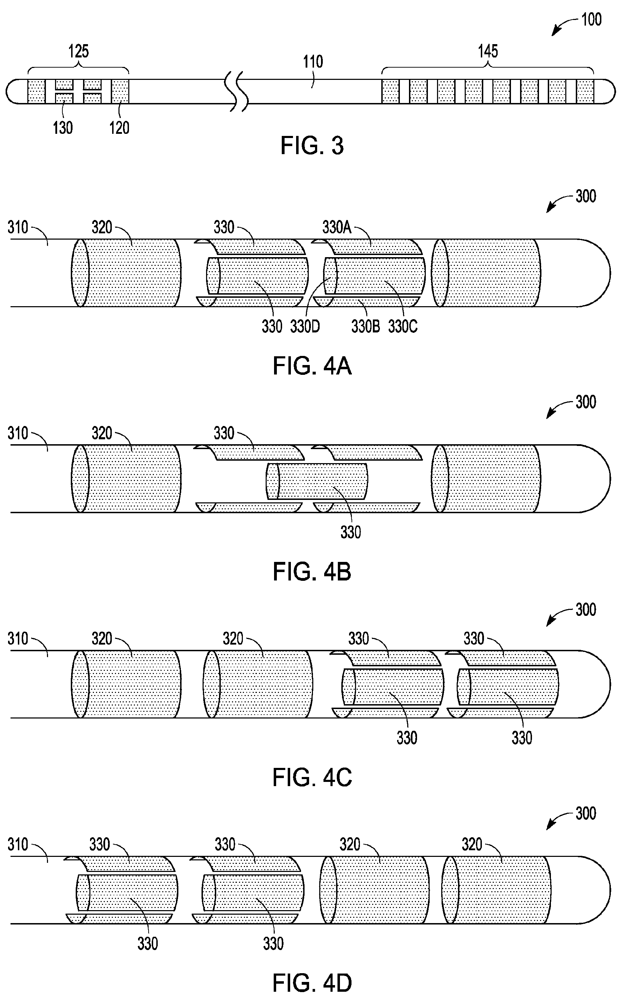

Figure 3 is a schematic side view of an embodiment of an electrical stimulation lead. Figure 3 illustrates a lead 110 with electrodes 125 disposed at least partially about a circumference of the lead 110 along a distal end portion of the lead and terminals 145 disposed along a proximal end portion of the lead. The electrodes can be used to spatially distribute fields of neurostimulation energy. The lead 110 can be implanted near or within the desired portion of the body to be stimulated (e.g., the brain, spinal cord, or other body organs or tissues). In one example of operation for deep brain stimulation, access to the desired position in the brain can be accomplished by drilling a hole in the patient's skull or cranium with a cranial drill (commonly referred to as a burr), and coagulating and incising the dura mater, or brain covering. The lead 110 can be inserted into the cranium and brain tissue with the assistance of a stylet (not shown). The lead 110 can be guided to the target location within the brain using, for example, a stereotactic frame and a microdrive motor system, optionally including cannulas. In some embodiments, the microdrive motor system can be fully or partially automatic. The microdrive motor system may be configured to perform one or more the following actions (alone or in combination): insert the lead 110, advance the lead 110, retract the lead 110, or rotate the lead 110.

In some embodiments, measurement devices coupled to the muscles or other tissues stimulated by the target neurons, or a unit responsive to the patient or clinician, can be coupled to the implantable pulse generator or microdrive motor system. The measurement device, user, or clinician can indicate a response by the target muscles or other tissues to the stimulation or recording electrode(s) to further identify the target neurons and facilitate positioning of the stimulation electrode(s). For example, if the target neurons are directed to a muscle experiencing tremors, a measurement device can be used to observe the muscle and indicate changes in, for example, tremor frequency or amplitude in response to stimulation of neurons. Alternatively, the patient or clinician can observe the muscle and provide feedback.

The lead 110 for deep brain stimulation can include stimulation electrodes, recording electrodes, or both. In at least some embodiments, the lead 110 is rotatable so that the stimulation electrodes can be aligned with the target neurons after the neurons have been located using the recording electrodes. Stimulation electrodes may be disposed on the circumference of the lead 110 to stimulate the target neurons. Stimulation electrodes may be ring-shaped so that current projects from each electrode equally in every direction from the position of the electrode along a length of the lead 110. In the embodiment of Figure 2, two of the electrodes are ring electrodes 120, 122. Ring electrodes typically do not enable stimulus current to be directed from only a limited angular range around of the lead. Segmented electrodes 130, 132, however, can be used to direct stimulus current to a selected angular range around the lead. When segmented electrodes are used in conjunction with an implantable pulse generator that delivers constant current stimulus with more than one independent stimulus source, current steering can be achieved to more precisely deliver the stimulus to a position along and around an axis of the lead (e.g., longitudinal and radial positioning around the axis of the lead). When current steering, segmented electrodes can be utilized in addition to, or as an alternative to, ring electrodes.

The lead 100 includes a lead body 110, terminals 145, and one or more ring electrodes 120, 122 and one or more sets of segmented electrodes 130, 132 (or any other combination of electrodes). The lead body 110 can be formed of a biocompatible, non-conducting material such as, for example, a polymeric material. Suitable polymeric materials include, but are not limited to, silicone, polyurethane, polyurea, polyurethaneurea, polyethylene, or the like. Once implanted in the body, the lead 100 may be in contact with body tissue for extended periods of time. In at least some embodiments, the lead 100 has a cross-sectional diameter of no more than 1.5 millimeters (1.5 mm) and may be in the range of 0.5 to 1. 5 mm. In at least some embodiments, the lead 100 has a length of at least 10 centimeters (10 cm) and the length of the lead 100 may be in the range of 10 to 70 cm.

The electrodes 125 can be made using a metal, alloy, conductive oxide, or any other suitable conductive biocompatible material. Examples of suitable materials include, but are not limited to, platinum, platinum iridium alloy, iridium, titanium, tungsten, palladium, palladium rhodium, or the like. Preferably, the electrodes are made of a material that is biocompatible and does not substantially corrode under expected operating conditions in the operating environment for the expected duration of use. Each of the electrodes can either be used or unused (OFF). When the electrode is used, the electrode can be used as an anode or cathode and carry anodic or cathodic current. In some instances, an electrode might be an anode for a period of time and a cathode for a period of time.

Deep brain stimulation leads and other leads may include one or more sets of segmented electrodes. Segmented electrodes may provide for finer current steering than ring electrodes because target structures in deep brain stimulation or other stimulation are not typically symmetric about the axis of the distal electrode array. Instead, a target may be located on one side of a plane running through the axis of the lead. Through the use of a radially segmented electrode array ("RSEA"), current steering can be performed not only along a length of the lead but also around a circumference of the lead. This provides precise three-dimensional targeting and delivery of the current stimulus to neural target tissue, while potentially avoiding stimulation of other non-target tissue.

Any number of segmented electrodes 130, 132 may be disposed on the lead body 110 including, for example, anywhere from one to sixteen or more segmented electrodes. It will be understood that any number of segmented electrodes may be disposed along the length of the lead body 110. A segmented electrode 130, 132 typically extends only 75%, 67%, 60%, 50%, 40%, 33%, 25%, 20%, 17%, 15%, or less around the circumference of the lead.

The segmented electrodes may be grouped into sets of segmented electrodes, where each set is disposed around a circumference of the lead 100 at a particular longitudinal portion of the lead 100. The lead 100 may have any number segmented electrodes in a given set of segmented electrodes. The lead 100 may have one, two, three, four, five, six, seven, eight, or more segmented electrodes in a given set. In at least some embodiments, each set of segmented electrodes of the lead 100 contains the same number of segmented electrodes. The segmented electrodes disposed on the lead 100 may include a different number of electrodes than at least one other set of segmented electrodes disposed on the lead 100. The segmented electrodes may vary in size and shape. In some embodiments, the segmented electrodes are all of the same size, shape, diameter, width or area or any combination thereof. In some embodiments, the segmented electrodes of each circumferential set (or even all segmented electrodes disposed on the lead 100) may be identical in size and shape.

Each set of segmented electrodes may be disposed around the circumference of the lead body 110 to form a substantially cylindrical shape around the lead body 110. The spacing between individual electrodes of a given set of the segmented electrodes may be the same, or different from, the spacing between individual electrodes of another set of segmented electrodes on the lead 100. In at least some embodiments, equal spaces, gaps or cutouts are disposed between each segmented electrode around the circumference of the lead body 110. In other embodiments, the spaces, gaps or cutouts between the segmented electrodes may differ in size, or cutouts between segmented electrodes may be uniform for a particular set of the segmented electrodes or for all sets of the segmented electrodes. The sets of segmented electrodes may be positioned in irregular or regular intervals along a length the lead body 110.

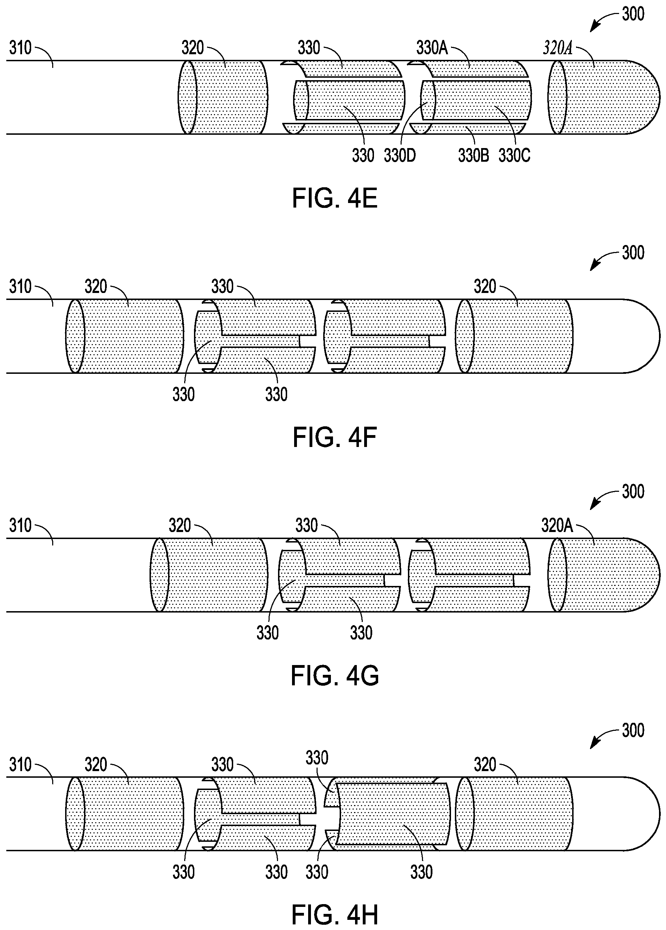

Conductor wires (not shown) that attach to the ring electrodes 120, 122 or segmented electrodes 130, 132 extend along the lead body 110. These conductor wires may extend through the material of the lead 100 or along one or more lumens defined by the lead 100, or both. The conductor wires couple the electrodes 120, 122, 130, 132 to the terminals 145. Figures 4A-4H are illustrations of different embodiments of leads 300 with segmented electrodes 330, optional ring electrodes 320 or tip electrodes 320a, and a lead body 310. The sets of segmented electrodes 330 each include either two (Figure 4B), three (Figures 4E-3H), or four (Figures 4A, 4C, and 4D) or any other number of segmented electrodes including, for example, three, five, six, or more. The sets of segmented electrodes 330 can be aligned with each other (Figures 4A-4G) or staggered (Figure 4H).

When the lead 100 includes both ring electrodes 120, 122 and segmented electrodes 130, 132, the ring electrodes and the segmented electrodes may be arranged in any suitable configuration. For example, when the lead 100 includes two ring electrodes and two sets of segmented electrodes, the ring electrodes can flank the two sets of segmented electrodes (see *e.g.*, Figures 3, 4A, and 4E-4H, ring electrodes 320 and segmented electrode 330). Alternately, the two sets of ring electrodes can be disposed proximal to the two sets of segmented electrodes (*see e.g.*, Figure 4C, ring electrodes 320 and segmented electrode 330), or the two sets of ring electrodes can be disposed distal to the two sets of segmented electrodes (*see e.g.*, Figure 4D, ring electrodes 320 and segmented electrode 330). One of the ring electrodes can be a tip electrode (see *e.g.*, tip electrode 320a of Figures 4E and 4G). It will be understood that other configurations are possible as well *(e.g*., alternating ring and segmented electrodes, or the like).

By varying the location of the segmented electrodes, different coverage of the target neurons may be selected. For example, the electrode arrangement of Figure 4C may be useful if the physician anticipates that the neural target will be closer to a distal tip of the lead body 110, while the electrode arrangement of Figure 4D may be useful if the physician anticipates that the neural target will be closer to a proximal end of the lead body 110.

Any combination of ring electrodes and segmented electrodes may be disposed on the lead 100. For example, the lead may include a first ring electrode, two sets of segmented electrodes; each set formed of four segmented electrodes, and a final ring electrode at the end of the lead. This configuration may simply be referred to as a 1-4-4-1 configuration (Figures 4A and 4E, ring electrodes 320 and segmented electrode 330). It may be useful to refer to the electrodes with this shorthand notation. Thus, the embodiment of Figure 4C may be referred to as a 1-1-4-4 configuration, while the embodiment of Figure 4D may be referred to as a 4-4-1-1 configuration. The embodiments of Figures 4F, 4G, and 4H can be referred to as a 1-3-3-1 configuration. Other electrode configurations include, for example, a 2-2-2-2 configuration, where four sets of segmented electrodes are disposed on the lead, and a 4-4 configuration, where two sets of segmented electrodes, each having four segmented electrodes are disposed on the lead. The 1-3-3-1 electrode configuration of Figures 4F, 4G, and 4H has two sets of segmented electrodes, each set containing three electrodes disposed around the circumference of the lead, flanked by two ring electrodes (Figures 4F and 4H) or a ring electrode and a tip electrode (Figure 4G). In some embodiments, the lead includes 16 electrodes. Possible configurations for a 16-electrode lead include, but are not limited to 4-4-4-4; 8-8; 3-3-3-3-3-1 (and all rearrangements of this configuration); and 2-2-2-2-2-2-2-2.

Any other suitable arrangements of segmented and/or ring electrodes can be used including, but not limited to, those disclosed in U.S. Provisional Patent Application Serial No. 62/113,291 and U.S. Patent Applications Publication Nos. 2012/0197375 and 2015/0045864. As an example, arrangements in which segmented electrodes are arranged helically with respect to each other. One embodiment includes a double helix. The leads and electrodes described in regarding to FIG. 1 through 4H are some examples of the types of leads and electrodes that can be used to implement the techniques described herein. Other arrangements of leads and electrodes can also be used.

One or more electrical stimulation leads can be implanted in the body of a patient (for example, in the brain or spinal cord of the patient) and used to stimulate surrounding tissue. The lead(s) are coupled to the implantable pulse generator (such as IPG 14 in Figure 1). After implantation, a clinician will program the IPG 14 using the clinician programmer, remote control, or other programming device. According to at least some programming techniques, the clinician enters stimulator parameters for a stimulation program and the stimulation program is used to stimulate the patient. The clinician observes the patient response. In at least some instances, the clinician asks the patient to describe, rate, or otherwise provide information about the effects of the stimulation such as what portion of the body is affected, how strong is the stimulation effect, whether there are side effects or negative effects, and the like.

Implantable neural stimulation electrodes may be implanted in a patient during surgery when the patient is mildly sedated or under general anesthesia. General anesthesia is a pharmacologically induced state of unresponsiveness and unconsciousness and is defined according to a set of clinical behavioral endpoints, such as a reversible state of hypnosis, analgesia, amnesia, and autonomic stability with good operating conditions. The depth of anesthesia of patient needs to be monitored at multiple stages of a surgical procedure. Currently, it is not fully understood how general anesthesia works on a system level neural basis. Current understanding of the neural activity during loss of consciousness (LOC) resulting from general anesthesia and subsequent recovery of consciousness (ROC) is incomplete. Deep brain structures such as those structures targeted in DBS (e.g., basal ganglia) may play a role in the LOC and ROC of a patient under general anesthesia, and hence monitoring neural activity of deep brain structures can help monitor depth of anesthesia of the patient. However, existing tools to monitor depth of anesthesia are mostly noninvasive and do not allow for observing neural activity of deep brain structures. Therefore, current techniques to monitor depth of anesthesia during surgical procedures may not fully apply to patients with neurological and neuropsychiatric disorders as disease that affects the brain may also affect a patient's response to anesthetic agents.

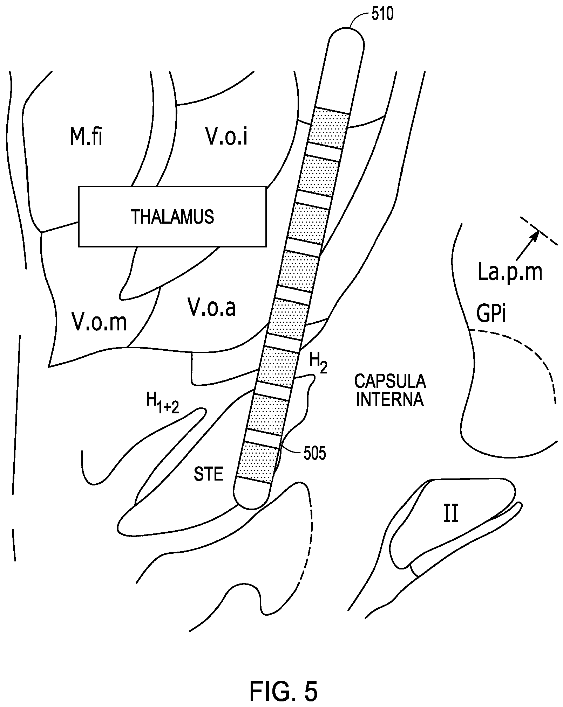

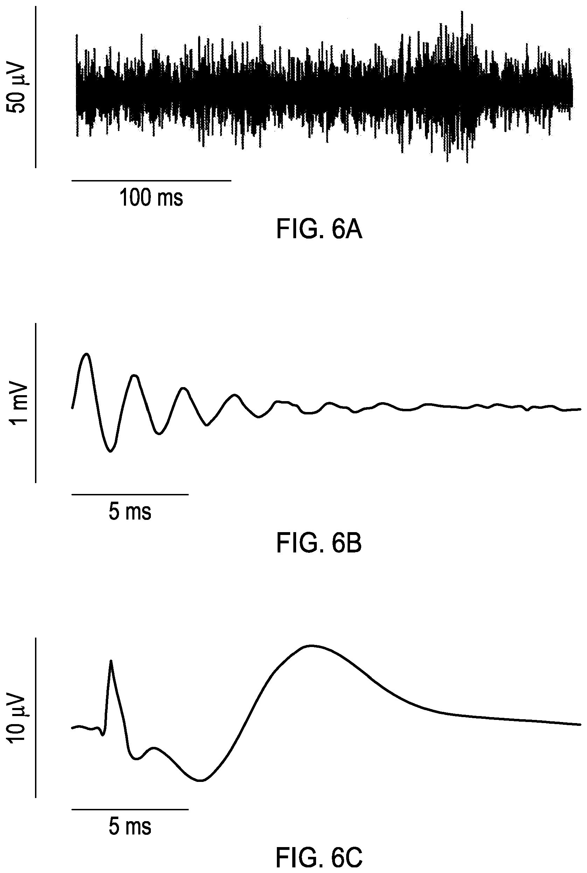

Implantable DBS electrodes can be used to sense neural signals as well as deliver neural stimulation energy. The neural signals of interest are local to deep brain structures rather than peripheral nerves that are located beyond the brain. Figure 5 is an illustration representing DBS electrode implantation on or near the subthalamic nucleus (STN) 505. The DBS electrodes of lead 510 are connected to a sensing circuit of an IPG 14 or ETS 20. The sensing circuit can be used to sense a spontaneous neural signal. Figure 6A is an illustration of an example of a spontaneous neural signal, such as a local field potential or other spontaneous neural activity.

In some examples, the DBS electrodes can also be connected to a stimulation circuit. The stimulation circuit may provide electrical neurostimulation. The stimulation circuit may be used to initiate an evoked neural signal that can be sensed using the sensing circuit. The sensed neural signal may be a local neural signal or a network neural signal. Heuristically, a 'local neural signal' refers to a signal that is generated from activity in the immediate neighborhood of the stimulating electrode, while a 'network neural signal' refers to a signal that is generated in or by the distributed brain networks to which the region of stimulation belongs.

Signals recorded at the site of stimulation (evoked or spontaneous) not only represent aspects of local neural activity but could be considered as a reflection of the ongoing transmission and dynamic flow of information across neural networks. It is useful to recognize that while the proportion of local and remote contributions at any site cannot be definitively ascertained from a single recording point, certain inferences regarding the source of various components may be possible from analysis of the neural data combined with knowledge of the connectivity across the network. Such inference is aided by recording signals synchronously from other brain areas remote to the site of stimulation.

For instance, Cortical evoked potentials generated by subthalamic DBS reflect the response of cortex to subcortical stimulation and are often recorded via paddle leads. Response from different neural substrates (local or network) appear in the recorded data in the form of amplitude peaks/troughs with variable time latencies. Research suggests that the short latency response may be due to antidromic activation of the hyperdirect pathway (cortex-STN). Long latency response may result from orthodromic propagation through basal ganglia-thalamus-cortex pathways, and additional pathways may be involved.

Figures 6B and 6C are illustrations of examples of evoked neural signals. Figure 6B illustrates an example of evoked resonant neural activity. An evoked resonant neural activity (ERNA) signal is a decaying oscillatory neural signal that can be sensed at various locations of the brain, including regions around the STN of the brain. Figure 6C illustrates another type of evoked neural response. A sensed evoked neural signal may be a local neural signal that is recorded at or near the site of neural stimulation. The evoked signal can be sensed using one or more electrodes different from the electrode or electrodes used to deliver the stimulation, or can be sensed using the same electrode used for the stimulation (or different segment of the same segmented electrode), including stimulation after a delay from the delivering the stimulation. Electrodes used for evoking the signals may be the same, a subset of, or distinct from the electrodes used for delivering neurostimulation therapy. A sensed evoked neural signal may be a network neural signal that is sensed in a different portion of a distributed brain network to which the brain region stimulated belongs.

Sensed neural signals, or derivatives of the neural signals, can be used as a biomarker to monitor depth of pharmacologically induced general anesthesia. General anesthesia may affect neural activity at deep brain structures, such as those that are often targeted by DBS. Investigating the signal characteristics of brain neural activity (such as ERNA signal waveforms) can assist in monitoring the depth of anesthesia, or assist in monitoring the effects of any medications that affect the arousal state of the patient or subject. Additionally, monitoring a neural signal such as an ERNA signal, or a derivative of an ERNA signal, can be used as an aid in placement of DBS electrodes.

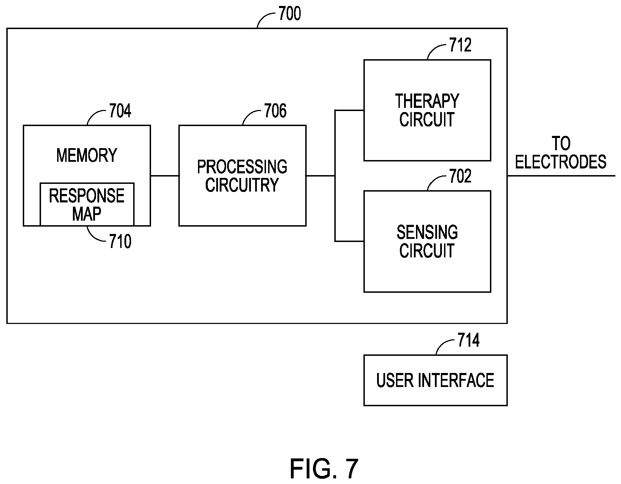

Figure 7 is a block diagram of an example of portions of a medical device 700 that monitors state of medication, depth of anesthesia, and the like, of a patient or subject. The medical device 700 may be an IPG 14 or an ETS 20. The medical device 700 includes a sensing circuit 702, a memory 704, and processing circuitry 706 in communication with the sensing circuit 702 and memory 704. The sensing circuit 702 may include one or more sense amplifiers that sense neural signals of a subject when the sensing circuit 702 is connected to implantable electrodes (e.g., implantable DBS electrodes). In some examples, the implantable electrodes are configured by shape and size for placement on or near a deep brain structure such as a basal ganglion of the brain or the STN of the brain. The medical device 700 can include a stimulation circuit 716 that delivers electrical neurostimulation when the stimulation circuit is connected to the implantable electrodes. The electrical neurostimulation by the stimulation circuit 716 may be used to initiate an evoked neural signal that can be sensed using the sensing circuit 702. The processing circuitry 706 can include a processor such as a microprocessor, a digital signal processor, application specific integrated circuit (ASIC), or other type of processor, interpreting or executing instructions in software modules or firmware modules. The memory 704 is connected to, or integral to, the processing circuitry 706.

The memory 704 stores one or more reference signals. The reference signals are representative of local or network level neural response to general anesthesia at or near a physiological location of an implantable electrode. For example, if the implantable electrode is positioned near or on the STN, the reference signals may be representative of the neural response to general anesthesia at the STN or at the broader basal ganglia-thalamocortical network. The stored reference signals may be features or characteristics of neural signals or other prior knowledge saved to memory for use in comparison to recorded signal or derivatives thereof. The reference signals can include baseline neural activity signals, or derivatives of the baseline neural activity signals. The reference signals may be obtained from single trials or by averaging groups of neural signals.

The stored reference signals can be waveforms corresponding to different predetermined depths of anesthesia. To determine the depth of anesthesia of the subject, the processing circuitry 706 quantifies the sensed neural signal, including comparison of the sensed neural signal to the reference signals. If the processing circuitry 706 determines that the sensed neural signal sufficiently matches a reference signal, the processing circuitry 706 may generate an indication (e.g., a confirmation signal or message) of the depth of anesthesia of the subject corresponding to the matched reference signal. If a match is not found, the indication may be presented to a user via a user interface 714 of an ETS 20, or the indication may be communicated from an IPG 14 to a CP 18, and the CP 18 presents the indication of the depth of anesthesia to the user via a user interface of the CP 18. For example, the reference signal may be either of the ERNA signal waveforms of Figure 6. If the processing circuitry matches a sensed ERNA signal waveform to a reference ERNA signal, the processing circuitry may indicate the depth of general anesthesia corresponding to the waveform, such as by presenting an indication of a stage of general anesthesia corresponding to the matched signal waveform. The processing circuitry 706 may present steps for the physician or clinician to take, such as steps to manage the depth of anesthesia for example.

The processing circuitry 706 may compare the determined depth of anesthesia to an expected depth of anesthesia. The expected depth of anesthesia may be input via the user interface or may be determined from other information (e.g., the general anesthesia drug type, the dose of the general anesthesia drug, the type of surgical procedure, etc.). If the determined depth of anesthesia is too different from the expected depth of anesthesia, the processing circuitry 706 may present a different indication to the user (e.g., a warning, message, or suggestion). Alternatively, the system may score the neural response, and present the score to the user for further interpretation.

According to some examples, the processing circuitry 706 may match a sensed neural signal and a reference signal by correlating the signals. For instance, the processing circuitry 706 determines correlation score associated with correlation of the morphology of the sensed neural signal to the morphology of a reference signal.

An example of a correlation score is a feature correlation coefficient (FCC). The FCC can provide an indication of a degree of similarity between the shape of the sensed neural signal and the shape of a reference signal. The one or more reference signals may be recorded for a particular subject or may be created based on a patient population. The correlation score can be applied to one or more time domain features of the sensed neural signal and the reference signal (e.g., peak amplitude), to one or more frequency domain features of the signals (e.g., energy of the signal at a specific frequency), or to a combination of time and frequency domain features of the signals.

According to some examples, the processing circuitry 706 performs a machine-learning algorithm to classify a sensed neural activity signal as matching a stored reference signal. The processing circuitry 706 may execute software that implements a machine-learning classifier that is trained to classify an input neural signal (e.g., a sensed neural signal) as a match to a stored reference signal. In some examples, the machine-learning classifier uses features of the signals to predict a match between signals. In some examples, the machine-learning classifier is a linear classifier that assigns a score to how well a sensed neural signal matches a stored reference signal and matches the sensed neural signal to a reference signal based on the score.

In some examples, the processing circuitry 706 implements a support vector machine (SVM) algorithm. The SVM algorithm may implement a machine-learning classifier that classifies the sensed neural signals using regression analysis. In some examples, the processing circuitry 706 implements decision tree learning. In decision tree learning, a decision tree is assigned nodes that are labeled with an input feature that can be a time domain or frequency domain feature of neural signals. Possible values of the features lead to subordinate decision nodes. Traversing the decision nodes leads to classification of the neural signal. Machine learning can change the weight or probability of the arcs between the decision nodes to skew decisions towards certain results.

The memory 704 may contain an anesthesia response map 710 that includes several reference signals indicative of anatomical locations of the brain. The anesthesia response map 710 may be indexed by anatomical location to identify a reference signal to compare to the sensed neural signal. The reference signal stored for a specific anatomical location may be a calibration of the expected neural signal for that location. Other auxiliary factors can be used to further index the stored neural reference signals. In some examples, the reference signals are further indexed using one or more of the type of general anesthetic drug, the dose of a general anesthetic drug, and the blood level concentration of the general anesthetic drug. In certain examples, the reference signals are further indexed using, but not limited to, the medical condition of the patient (e.g., movement disorder, chronic pain, traumatic brain injury, stroke, etc.), the disease state of the patient (e.g., Alzheimer's disease, etc.), and the medication state of the patient.

To generate the mapping database, one or more of knowledge of the surgical target, the anatomy of interest may be used. Also, the recorded reference may have been sensed using probe electrodes or DBS electrodes, and the stored reference signals may be stored in association with one or more of which type of evoking or recording electrode is used, where the electrode is placed, and the parameters used when evoking the reference signals.

In some examples, the medical device 700 includes a therapy circuit 712. The therapy circuit 712 may control dosing of the general anesthesia drug or general anesthetic agent. In some examples, the medical device is an external device, and the therapy circuit 712 electrically activates a valve to dispense the drug from a drug reservoir. The processing circuitry 706 adjusts delivery of the general anesthetic drug according to the indication of depth of anesthesia of the subject. This activation can implement closed loop control over the dispensing of the general anesthesia drug.

It can be seen from the devices and techniques described herein that sensed neural signals can be used as a biomarker to monitor depth of pharmacologically induced general anesthesia of a patient or subject and the monitoring can be used to control the depth of the anesthesia. Sensed neural signals can also be used as a biomarker to aid in the placement of implantable electrodes.

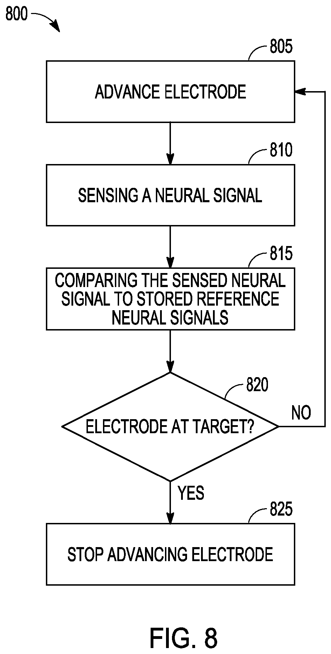

Figure 8 is a flow diagram of an example of a method 800 of DBS implantable lead placement. At 805, the lead is advanced toward a target anatomical location within the brain. The lead may be advanced to the target location within the brain using, for example, a stereotactic frame and a microdrive motor system, and signal processing during the procedure can be performed using the medical device 700 in Figure 7. At 810, a neural signal is sensed using lead electrodes and the sensing circuit 702, and the neural signal may be recorded or stored in memory 704.

The anesthesia response map 710 can include multiple reference signals that are each representative of a local response to general anesthesia at or near a specific anatomical location. The reference signals may include baseline network evoked or spontaneous neural signals or derivatives of baseline network evoked or spontaneous neural signals. The reference signals may be patient-specific or determined from a patient population. In certain examples, the reference signals are disease specific. The dependence of the reference signals on brain location may be determined using imaging data or other neurophysiological signals commonly acquired during the surgical procedure. During the surgical procedure to place implantable electrodes, at block 815 the processing circuitry 706 searches the anesthesia response map by comparing the sensed neural signal to the stored reference signals. When the processing circuitry 706 matches the sensed neural signal to a stored reference signal, the lead electrode may be at the anatomical location associated with the identified reference signal. For example, the stored reference signals may be reference ERNA signals and the target location may be the STN of the brain. The processing circuitry 706 may determine that the lead electrode is at the STN when the sensed ERNA signal matches the reference ERNA signal for the STN.

Different techniques may be used to search the stored reference signals for a match of the sensed neural signal or derivatives thereof. In certain examples, the processing circuitry implements a machine learning algorithm or product of a machine learning process to match sensed neural signals to stored reference signals. The machine learning algorithm may be a support vector machine algorithm, a decision tree algorithm, or may include a machine classifier such as a linear classifier or a regression classifier. In certain examples, template matching using correlation is used to match sensed neural signals to stored reference signals, such as by matching one or more time domain features or frequency domain features of the sensed neural signal and the stored reference signals. The searching algorithm may start the search with the last location of the electrode and proceed with first checking the nearest locations for a matching neural signal.

When a matching neural signal is found, the processing circuitry 706 may generate an indication of the specific anatomical location corresponding to the reference signal as the location of the implantable electrode. For example, medical device may be an ETS 20, and the identified anatomical location may be presented to a user via a user interface 714 of the ETS 20. In another example, the medical device 700 communicates the anatomical location (by wired or wireless means) to a separate device and the anatomical location is presented to the user via a user interface of the separate device. In this way the position of the electrodes being placed can be monitored and communicated during the surgical procedure.

At block 820, the method 800 can include determining when the electrode or electrodes are positioned at the target anatomical location. The medical device 700 may be an external device and include a user interface 714. The processing circuitry 706 receives a target location of the brain and determines the reference signal for the target location. As the implantable lead is advanced, the sensing circuit 702 senses neural signals and the processing circuitry 706 determines if the sensed neural signals match the reference signal for the target location. When the sensed signal matches the reference signal, the implantable electrode is positioned at the target location and lead advancement stops at 825.

The processing circuitry 706 may generate an indication via the user interface 714 that the implantable electrode is positioned at the target location. In certain examples, the medical device 700 does not include a user interface and the target location is received from a separate device which has the user interface 714. The medical device 700 sends the indication that the electrode is positioned at the target location to the separate device which notifies the user that the lead electrode is positioned at the target location. In certain examples, medical device 700 provides confirmation via the user interface that the lead is positioned in the correct location of the brain. In certain examples, the medical device 700 provides information about which electrodes or segment(s) of a segmented electrode to use to provide electrical neurostimulation energy or to use to sense neural signals.

The medical device 700 may iteratively compare the sensed neural signal to the reference signal of the target as the lead is advanced. In some examples, as the lead is advanced, the processing circuitry 706 determines the relative position of the implantable electrode and the target location. For instance, the processing circuitry may find a reference signal that matches the sensed neural signal during advancement of the lead, but the matching signal is not the reference signal of the target location. The processing circuitry 706 may use the anatomical location associated with the matching reference signal to indicate the relative position of the lead electrode to the target location.

As shown in the wave forms of Figure 6, the neural signals may change according to the depth of anesthesia of the patient or subject. Because of this, it may be useful for the anesthesia map to include multiple reference signals for an anatomical location with the multiple signals representing different depths of anesthesia. The reference signals may be stored in the anesthesia response map 710 in association with anatomical location and one or both of the general anesthesia drug type and the dose of the general anesthesia. The processing circuitry 706 may receive the drug type and the dose at the beginning of the procedure and during the procedure. To determine the reference signal to use for comparing to the sensed neural signal, the processing circuitry 706 searches the reference signals in the anesthesia response map 710 using one or both of the type of anesthesia drug and the dose of the anesthesia drug.

Matching the neural signals may provide information about the arousal state of the patient. In some examples, the medical device 700 may modify the anesthesia during placement of the lead. For instance, the processing circuitry 706 may modify dosing by the therapy circuit 712 to maintain a specific depth of anesthesia of the patient. In another example, the processing circuitry 706 may lighten the anesthesia dose delivered by the therapy circuit 712 to enhance recording of signals to confirm lead placement, or to bring the patient to a state where functional testing of neural stimulation better reflects the chronic response to the stimulation.

In some examples, the processing circuitry 706 receives anesthesia state information via the user interface regarding an anesthesia state of the subject. This information may be based on observations of a clinician or anesthesiologist. The processing circuitry 706 may compare the depth of anesthesia indicated in the sensed neural signal (and optionally the reference signal) to the received anesthesia state information. If the depth of anesthesia of the signal or signals is not consistent with the received anesthesia state information, the processing circuitry may generate an indication of the inconsistency. The indication may be in the form of an alert presented via a user interface.

The devices, systems and methods described herein use a sensed neural signal as a biomarker of depth of pharmacologically induced general anesthesia. This biomarker can be used to monitor depth of anesthesia of a patient or subject during a clinical or surgical procedure. The biomarker can also provide anatomical location information when implanting electrodes.

The embodiments described herein can be methods that are machine or computer-implemented at least in part. Some embodiments may include a computer-readable medium or machine-readable medium encoded with instructions operable to configure an electronic device or system to perform methods as described in the above examples. An implementation of such methods can include code, such as microcode, assembly language code, a higher-level language code, or the like. Such code can include computer readable instructions for performing various methods. The code can form portions of computer program products. Further, the code can be tangibly stored on one or more volatile or non-volatile computer-readable media during execution or at other times. Various embodiments are illustrated in the figures above. One or more features from one or more of these embodiments may be combined to form other embodiments.

The above detailed description is intended to be illustrative, and not restrictive. The scope of the present invention is defined by the appended claims.

## Claims

1. A medical system for coupling to one or more implantable electrodes, the system comprising:
a sensing circuit (702) configured to sense one or more neural signals representative of neural activity of a subject when connected to an implantable electrode of the one or more implantable electrodes;
a memory (704) to store a reference signal, wherein the reference signal is representative of a neural response associated with a state of arousal at or near a location of the implantable electrode; and
processing circuitry (706) in communication with the sensing circuit and the memory, the processing circuitry configured to:
compare the one or more sensed neural signals to the reference signal including comparing the one or more sensed neural signals to multiple stored reference signals, each reference signal associated with an anatomical location and representative of a local and network response to general anesthesia at or near the anatomical location, and matching the one or more sensed neural signals to a reference signal of the multiple stored reference signals; and
determine a depth of anesthesia of the subject according to the comparison of the one or more sensed neural signals and the reference signal; and
generate an indication of the anatomical location corresponding to the reference signal as the location of the implantable electrode.

2. The system of claim 1, including:
a stimulation circuit configured to deliver electrical neurostimulation to initiate an evoked neural signal, and
wherein the sensing circuit is configured to sense the evoked neural signal at or near the location of the implantable electrode, and the reference signal is a baseline evoked resonant neural activity signal for a specific anatomical location of the subject.

3. The system of claim 1 or 2, including:
a stimulation circuit configured to deliver electrical neurostimulation to initiate an evoked neural signal, and
wherein the sensing circuit is configured to sense a local evoked neural signal at or near a location of the electrical neurostimulation, and the reference signal is a baseline local evoked neural signal for a specific anatomical location of the subject.

4. The system of any one of claims 1-3, including:
a stimulation circuit configured to deliver electrical neurostimulation to initiate an evoked neural signal, and
wherein the sensing circuit is configured to sense a network evoked neural signal using the implantable electrode, and the reference signal is a baseline network evoked neural signal for a specific anatomical location of the subject.

5. The system of any one of claims 1-4, including the implantable electrode, and the implantable electrode is configured for placement at or near a basal ganglia structure of the subject.

6. The system of any one of claims 1-5, wherein the sensing circuit is configured to sense a spontaneous neural activity signal, and the reference signal is a baseline spontaneous neural activity signal associated with the state of arousal.

7. The system of any one of claims 1-6, including:
a therapy circuit configured to control dosing of the subject with a general anesthesia drug; and
wherein the processing circuitry is configured to adjust delivery of the general anesthesia drug according to the indication of depth of anesthesia of the subject.

8. The system of any one of claims 1-7,
wherein the memory stores an anesthesia response map that contains reference signals stored in association with anatomical locations and with one or more of a type of general anesthetic drug and a dose of a general anesthetic drug; and
wherein the processing circuit is configured to:
compare the one or more sensed neural signals to the reference signal associated with an anatomical location of the implantable electrode; and
generate the indication of depth of anesthesia of the subject using the comparison to the reference signal, and the one or more of the type of general anesthetic drug and the dose of general anesthetic drug.

9. The system of any one of claims 1-8, wherein the processing circuitry is configured to:
determine a measure of association of one or more time domain features of the one or more sensed neural signals to one or more time domain features of the reference signal; and
generate the indication of depth of anesthesia of the subject according to the determined correlation.

10. The system of any one of claims 1-9, wherein the processing circuitry is configured to:
determine a measure of association of one or more frequency domain features of the one or more sensed neural signals to one or more frequency domain features of the reference signal; and
generate the indication of depth of anesthesia of the subject according to the determined correlation.

11. The system of any one of claims 1-10, wherein the processing circuitry is configured to perform a machine learning algorithm to classify the one or more sensed neural activity signals as matching the reference signal.

12. A method of operating a medical system, the method comprising:
sensing one or more neural signals representative of neural activity of a subject using the medical system;
comparing the one or more sensed neural signals to a reference signal using the medical system, wherein the reference signal is representative of a neural response associated with a state of arousal at or near an anatomical location of an implantable electrode, wherein the comparing the one or more sensed neural signals to a reference signal includes comparing the one or more sensed neural signals to multiple stored reference signals, each reference signal associated with an anatomical location and representative of a local and network response to general anesthesia at or near the anatomical location; and matching the one or more sensed neural signals to a reference signal of the multiple stored reference signals; and
determining a depth of anesthesia of the subject according to the comparison of the one or more sensed neural signals and the reference signal;
generating an indication of the anatomical location corresponding to the reference signal as the location of the implantable electrode.

13. The method of claim 12, including:
receiving a target anatomical location via a user interface of the medical system;
matching the one or more sensed neural signals to a reference signal for the target location; and
generating an indication via the user interface that the implantable electrode is positioned at the target anatomical location.

14. The method of claim 12 or 13, including:
receiving a target anatomical location via a user interface of the medical system;
determining an anatomical location of the implantable electrode by matching the one or more sensed neural signals to the reference signal; and
indicating relative position of the implantable electrode and the target anatomical location via the user interface.

## Patentansprüche

1. Medizinisches System zum Koppeln mit einer oder mehreren implantierbaren Elektroden, wobei das System aufweist:
eine Erfassungsschaltung (702), die konfiguriert ist, ein oder mehrere neurale Signale zu erfassen, die für die neurale Aktivität eines Probanden repräsentativ sind, wenn sie mit einer implantierbaren Elektrode der einen oder mehreren implantierbaren Elektroden verbunden ist;
einen Speicher (704), um ein Referenzsignal zu speichern, wobei das Referenzsignal repräsentativ für eine neuronale Reaktion ist, die mit einem Erregungszustand an oder in der Nähe einer Stelle der implantierbaren Elektrode verbunden ist; und
eine Verarbeitungsschaltung (706), die mit der Erfassungsschaltung und dem Speicher in Verbindung steht, wobei die Verarbeitungsschaltung konfiguriert ist, um:
das eine oder die mehreren erfassten neuronalen Signale mit dem Referenzsignal zu vergleichen, was das Vergleichen des einen oder der mehreren erfassten neuronalen Signale mit mehreren gespeicherten Referenzsignalen, wobei jedes Referenzsignal mit einem anatomischen Ort assoziiert ist und repräsentativ für eine lokale und Netzwerkreaktion auf eine Allgemeinanästhesie an oder nahe des anatomischen Orts ist, und das Anpassen des einen oder der mehreren erfassten neuronalen Signale an ein Referenzsignal der mehreren gespeicherten Referenzsignale umfasst; und
eine Tiefe der Anästhesie des Probanden nach dem Vergleich des einen oder der mehreren erfassten neuronalen Signale und des Referenzsignals zu bestimmen; und
eine Anzeige des anatomischen Orts, der dem Referenzsignal entspricht, als den Ort der implantierbaren Elektrode zu bestimmen.

2. System nach Anspruch 1, das aufweist:
eine Stimulationsschaltung, die konfiguriert ist, eine elektrische Neurostimulation abzugeben, um ein evoziertes neuronales Signal auszulösen, und
wobei die Erfassungsschaltung konfiguriert ist, das evozierte neurale Signal an oder in der Nähe des Ortes der implantierbaren Elektrode zu erfassen, und das Referenzsignal ein Grundliniensignal der evozierten resonanten neuralen Aktivität für einen spezifischen anatomischen Ort des Probanden ist.

3. System nach Anspruch 1 oder 2, das aufweist:
eine Stimulationsschaltung, die konfiguriert ist, eine elektrische Neurostimulation abzugeben, um ein evoziertes neurales Signal auszulösen, und
wobei die Erfassungsschaltung konfiguriert ist, ein lokales evoziertes neurales Signal an oder in der Nähe eines Ortes der elektrischen Neurostimulation zu erfassen, und das Referenzsignal ein lokales evoziertes neurales Grundliniensignal für einen bestimmten anatomischen Ort des Probanden ist.

4. System nach einem der Ansprüche 1 bis 3, die aufweist:
eine Stimulationsschaltung, die konfiguriert ist, sie eine elektrische Neurostimulation abzugeben, um ein evoziertes neurales Signal auszulösen, und
wobei die Erfassungsschaltung konfiguriert ist, ein evoziertes neuronales Netzwerk-Signal unter Verwendung der implantierbaren Elektrode zu erfassen, und das Referenzsignal ein evoziertes neuronales Grundlinien-Netzwerk-Signal für einen bestimmten anatomischen Ort des Probanden ist.

5. System nach einem der Ansprüche 1 bis 4, das die implantierbare Elektrode aufweist, und die implantierbare Elektrode zur Platzierung an oder in der Nähe einer Basalganglienstruktur des Probanden konfiguriert ist.

6. System nach einem der Ansprüche 1 bis 5, wobei die Erfassungsschaltung konfiguriert ist, ein spontanes neuronales Aktivitätssignal zu erfassen, und das Referenzsignal ein spontanes neuronales Grundlinien-Aktivitätssignal ist, das mit dem Erregungszustand verbunden ist.

7. System nach einem der Ansprüche 1 bis 6, die aufweist:
eine Therapieschaltung, die konfiguriert ist, die Dosierung des Probanden mit einem Allgemeinanästhesie-Medikament zu steuern; und
wobei die Verarbeitungsschaltung konfiguriert ist, die Abgabe des Allgemeinanästhesie-Medikaments gemäß der Anzeige der Tiefe der Anästhesie des Probanden anzupassen.

8. System nach einem der Ansprüche 1 bis 7,
wobei der Speicher eine Anästhesie-Reaktionskarte speichert, die Referenzsignale enthält, die in Verbindung mit anatomischen Orten und mit einer oder mehreren eines Typs eines Allgemeinanästhesie-Medikaments und einer Dosis eines Allgemeinanästhesie-Medikaments gespeichert sind; und
wobei die Verarbeitungsschaltung konfiguriert ist, um:
das eine oder die mehreren erfassten neuralen Signale mit dem Referenzsignal zu vergleichen, das mit einem anatomischen Ort der implantierbaren Elektrode assoziiert ist; und
die Anzeige der Tiefe der Anästhesie des Probanden unter Verwendung des Vergleichs mit dem Referenzsignal und dem einen oder mehreren des Typs des Allgemeinanästhesie-Medikaments und der Dosis des Allgemeinanästhesie-Medikaments zu erzeugen.

9. System nach einem der Ansprüche 1 bis 8, wobei die Verarbeitungsschaltung konfiguriert ist, um:
ein Maß der Assoziation von einem oder mehreren Zeitbereichsmerkmalen des einen oder der mehreren erfassten neuralen Signale mit einem oder mehreren Zeitbereichsmerkmalen des Referenzsignals zu bestimmen; und
die Anzeige der Tiefe der Anästhesie des Probanden gemäß der bestimmten Korrelation zu erzeugen.

10. System nach einem der Ansprüche 1 bis 9, wobei die Verarbeitungsschaltung konfiguriert ist, um:
ein Maß der Assoziation von einem oder mehreren Frequenzbereichsmerkmalen des einen oder der mehreren erfassten neuralen Signale mit einem oder mehreren Frequenzbereichsmerkmalen des Referenzsignals zu bestimmen; und
die Anzeige der Tiefe der Anästhesie des Probanden gemäß der bestimmten Korrelation zu erzeugen

11. System nach einem der Ansprüche 1 bis 10, wobei die Verarbeitungsschaltung konfiguriert ist, einen maschinellen Lernalgorithmus durchzuführen, um das eine oder die mehreren erfassten neuronalen Aktivitätssignale als mit dem Referenzsignal übereinstimmend zu klassifizieren.

12. Verfahren zum Betreiben eines medizinischen Systems, wobei das Verfahren aufweist:
Erfassen eines oder mehrerer neuronaler Signale, die für die neuronale Aktivität eines Probanden repräsentativ sind, unter Verwendung des medizinischen Systems;
Vergleichen des einen oder der mehreren erfassten neuronalen Signale mit einem Referenzsignal unter Verwendung des medizinischen Systems, wobei das Referenzsignal für eine neuronale Reaktion repräsentativ ist, die mit einem Erregungszustand an oder in der Nähe eines anatomischen Ortes einer implantierbaren Elektrode verbunden ist, wobei das Vergleichen des einen oder der mehreren erfassten neuronalen Signale mit einem Referenzsignal das Vergleichen des einen oder der mehreren erfassten neuronalen Signale mit mehreren gespeicherten Referenzsignalen umfasst, wobei jedes Referenzsignal mit einem anatomischen Ort assoziiert ist und für eine lokale und Netzwerkreaktion auf eine Allgemeinanästhesie an oder in der Nähe des anatomischen Ortes repräsentativ ist; und Abgleichen des einen oder der mehreren erfassten neuronalen Signale mit einem Referenzsignal der mehreren gespeicherten Referenzsignale; und
Bestimmen einer Tiefe der Anästhesie des Probanden gemäß dem Vergleich des einen oder der mehreren erfassten neuronalen Signale und des Referenzsignals;
Erzeugen einer Anzeige für den anatomischen Ort, der dem Referenzsignal entspricht, als den Ort der implantierbaren Elektrode.

13. Verfahren nach Anspruch 12, das aufweist:
Empfangen eines anatomischen Zielortes über eine Benutzerschnittstelle des medizinischen Systems;
Abgleichen des einen oder der mehreren erfassten neuronalen Signale mit einem Referenzsignal für den Zielort; und
Erzeugen einer Anzeige über die Benutzerschnittstelle, dass die implantierbare Elektrode an dem anatomischen Zielort positioniert ist.

14. Verfahren nach Anspruch 12 oder 13, das aufweist:
Empfangen eines anatomischen Zielortes über eine Benutzerschnittstelle des medizinischen Systems;
Bestimmen eines anatomischen Ortes der implantierbaren Elektrode durch Abgleichen des einen oder der mehreren erfassten neuralen Signale mit dem Referenzsignal; und
Anzeigen der relativen Position der implantierbaren Elektrode und des anatomischen Zielortes über die Benutzerschnittstelle.

## Revendications

1. Système médical destiné à être couplé à une ou plusieurs électrodes implantables, le système comprenant :
un circuit de détection (702) configuré pour détecter un ou plusieurs signaux neuronaux représentatifs de l'activité neuronale d'un sujet lorsqu'il est connecté à une électrode implantable des une ou plusieurs électrodes implantables ;
une mémoire (704) destinée à stocker un signal de référence, dans lequel le signal de référence est représentatif d'une réponse neuronale associée à un état d'éveil au niveau ou à proximité d'un emplacement de l'électrode implantable ; et
des circuits de traitement (706) en communication avec le circuit de détection et la mémoire, les circuits de traitement étant configurés pour :
comparer les un ou plusieurs signaux neuronaux détectés avec le signal de référence, y compris en comparant les un ou plusieurs signaux neuronaux détectés avec plusieurs signaux de référence stockés, chaque signal de référence étant associé à un emplacement anatomique et représentatif d'une réponse locale et d'une réponse du réseau neuronal à une anesthésie générale au niveau ou à proximité de l'emplacement anatomique, et en mettant en correspondance les un ou plusieurs signaux neuronaux détectés avec un signal de référence des multiples signaux de référence stockés ; et
déterminer une profondeur d'anesthésie du sujet selon la comparaison entre les un ou plusieurs signaux neuronaux détectés et le signal de référence ;
et
générer une indication de l'emplacement anatomique qui correspond au signal de référence comme étant l'emplacement de l'électrode implantable.

2. Système selon la revendication 1, comprenant :
un circuit de stimulation configuré pour délivrer une neurostimulation électrique afin de déclencher un signal neuronal provoqué, et
dans lequel le circuit de détection est configuré pour détecter le signal neuronal provoqué au niveau ou à proximité de l'emplacement de l'électrode implantable, et le signal de référence est un signal d'activité neuronale résonant provoqué de référence pour un emplacement anatomique spécifique du sujet.

3. Système selon la revendication 1 ou 2, comprenant :
un circuit de stimulation configuré pour délivrer une neurostimulation électrique afin de déclencher un signal neuronal provoqué, et
dans lequel le circuit de détection est configuré pour détecter un signal neuronal provoqué local au niveau ou à proximité d'un emplacement de la neurostimulation électrique, et le signal de référence est un signal neuronal provoqué local de référence pour un emplacement anatomique spécifique du sujet.

4. Système selon l'une quelconque des revendications 1 à 3, comprenant :
un circuit de stimulation configuré pour délivrer une neurostimulation électrique afin de déclencher un signal neuronal provoqué, et
dans lequel le circuit de détection est configuré pour détecter un signal neuronal provoqué de réseau à l'aide de l'électrode implantable, et le signal de référence est un signal neuronal provoqué de réseau de référence pour un emplacement anatomique spécifique du sujet.

5. Système selon l'une quelconque des revendications 1 à 4, comprenant l'électrode implantable, et dans lequel l'électrode implantable est configurée pour être placée au niveau ou à proximité d'une structure de noyaux gris centraux du sujet.

6. Système selon l'une quelconque des revendications 1 à 5, dans lequel le circuit de détection est configuré pour détecter un signal d'activité neuronale spontanée, et le signal de référence est un signal d'activité neuronale spontanée de référence associé à l'état d'éveil.

7. Système selon l'une quelconque des revendications 1 à 6, comprenant :
un circuit de thérapie configuré pour contrôler le dosage du sujet avec un produit d'anesthésie générale ; et
dans lequel les circuits de traitement sont configurés pour ajuster l'administration du produit d'anesthésie générale selon l'indication de profondeur d'anesthésie du sujet.

8. Système selon l'une quelconque des revendications 1 à 7,
dans lequel la mémoire stocke une cartographie de réaction à l'anesthésie qui contient des signaux de référence stockés en association avec les emplacements anatomiques et avec un ou plusieurs d'un type de produit d'anesthésie générale et d'une dose de produit d'anesthésie générale ;
et
dans lequel le circuit de traitement est configuré pour :
comparer les un ou plusieurs signaux neuronaux détectés avec le signal de référence associé à un emplacement anatomique de l'électrode implantable ; et
générer l'indication de profondeur d'anesthésie du sujet à l'aide de la comparaison avec le signal de référence, et de l'un ou plusieurs du type de produit d'anesthésie générale et de la dose de produit d'anesthésie générale.

9. Système selon l'une quelconque des revendications 1 à 8, dans lequel les circuits de traitement sont configurés pour :
déterminer une mesure d'association d'une ou plusieurs caractéristiques de domaine temporel des un ou plusieurs signaux neuronaux détectés à une ou plusieurs caractéristiques de domaine temporel du signal de référence ; et
générer l'indication de profondeur d'anesthésie du sujet selon la corrélation déterminée.

10. Système selon l'une quelconque des revendications 1 à 9, dans lequel les circuits de traitement sont configurés pour :
déterminer une mesure d'association d'une ou plusieurs caractéristiques de domaine de fréquences des un ou plusieurs signaux neuronaux détectés à une ou plusieurs caractéristiques de domaine de fréquences du signal de référence ; et
générer l'indication de profondeur d'anesthésie du sujet selon la corrélation déterminée.

11. Système selon l'une quelconque des revendications 1 à 10, dans lequel les circuits de traitement sont configurés pour exécuter un algorithme d'apprentissage machine afin de classifier les un ou plusieurs signaux d'activité neuronale détectés comme correspondant au signal de référence.

12. Procédé de fonctionnement d'un système médical, le procédé comprenant :
la détection d'un ou plusieurs signaux neuronaux représentatifs de l'activité neuronale d'un sujet à l'aide du système médical ;
la comparaison des un ou plusieurs signaux neuronaux détectés avec un signal de référence à l'aide du système médical, dans lequel le signal de référence est représentatif d'une réponse neuronale associée à un état d'éveil au niveau ou à proximité d'un emplacement anatomique d'une électrode implantable, dans lequel la comparaison des un ou plusieurs signaux neuronaux détectés avec un signal de référence comprend la comparaison des un ou plusieurs signaux neuronaux détectés avec plusieurs signaux de référence stockés, chaque signal de référence étant associé à un emplacement anatomique et représentatif d'une réponse locale et d'une réponse de réseau neuronal à une anesthésie générale au niveau ou à proximité de l'emplacement anatomique ; et la mise en correspondance des un ou plusieurs signaux neuronaux détectés avec un signal de référence des multiples signaux de référence stockés ; et
la détermination d'une profondeur d'anesthésie du sujet selon la comparaison des un ou plusieurs signaux neuronaux détectés et du signal de référence ;
la génération d'une indication de l'emplacement anatomique qui correspond au signal de référence comme étant l'emplacement de l'électrode implantable.

13. Procédé selon la revendication 12, comprenant :
la réception d'un emplacement anatomique cible via une interface utilisateur du système médical ;
la mise en correspondance des un ou plusieurs signaux neuronaux détectés avec un signal de référence pour l'emplacement cible ; et
la génération d'une indication, via l'interface utilisateur, selon laquelle l'électrode implantable est positionnée au niveau de l'emplacement anatomique cible.

14. Procédé selon la revendication 12 ou 13, comprenant :
la réception d'un emplacement anatomique cible via une interface utilisateur du système médical ;
la détermination d'un emplacement anatomique de l'électrode implantable en mettant en correspondance les un ou plusieurs signaux neuronaux détectés avec le signal de référence ; et
l'indication de la position relative de l'électrode implantable et de l'emplacement anatomique cible via l'interface utilisateur.
